# EUROPÄISCHE PATENTSCHRIFT

(11) **EP 4 622 566 B1**
(45) Veröffentlichungstag und Bekanntmachung des Hinweises auf die Patenterteilung: **12.08.2026**
(21) Anmeldenummer: 24836966.2
(22) Anmeldetag: 18.12.2024
(51) Int. Cl.: A61B 17/29, A61B 18/14

(54) **BETÄTIGUNGSEINRICHTUNG FÜR EIN MEDIZINISCHES INSTRUMENT UND MEDIZINISCHES INSTRUMENT UMFASSEND EINE SOLCHE BETÄTIGUNGSEINRICHTUNG**
ACTUATION DEVICE FOR A MEDICAL INSTRUMENT, AND MEDICAL INSTRUMENT COMPRISING SUCH AN ACTUATION DEVICE
DISPOSITIF D'ACTIONNEMENT POUR UN INSTRUMENT MEDICAL, ET INSTRUMENT MEDICAL COMPRENANT UN TEL DISPOSITIF D'ACTIONNEMENT

(30) Priorität: 21.12.2023 DE 102023136224
(43) Veröffentlichungstag der Anmeldung: 01.10.2025
(73) Patentinhaber: Aesculap AG, 78532 Tuttlingen (DE)
(72) Erfinder: SEYFRIED, Dominik, 78126 Königsfeld (DE); KIZENBERGER, Hannes, 78194 Immendingen (DE)
(74) Vertreter: DREISS Patentanwälte PartG mbB
(86) Internationale Anmeldenummer: PCT/EP2024/087301
(87) Internationale Veröffentlichungsnummer: WO 2025/132717

(56) Entgegenhaltungen:
- WO-A1-2022/171668
- US-A1- 2015 073 394
- US-B1- 6 443 968

## Beschreibung

Die Offenbarung betrifft eine Betätigungseinrichtung für ein medizinisches Instrument und ein medizinisches Instrument mit einer Betätigungseinrichtung. Bei einem solchen medizinischen Instrument handelt es sich beispielsweise um ein chirurgisches, insbesondere elektrochirurgisches Instrument, beispielsweise ein Gefäßversiegelungsinstrument, insbesondere zur bipolaren Gefäßversiegelung.

In etwa der offenen und laparoskopischen Chirurgie sind beispielsweise in den chirurgischen Disziplinen der Allgemeinchirurgie, der Gynäkologie, der Urologie und der Thoraxchirurgie Geräte oder Systeme zur bipolaren Gefäßversiegelung bekannt.

So ist beispielsweise aus US 6,443,968 B1 ein chirurgisches Instrument, wie beispielsweise ein ultraschallbetriebenes chirurgisches Schneid- und Koagulationsgerät, bekannt, bei dem die Greifbewegung des Chirurgen mechanisch in eine laterale Bewegung zum Betätigen beispielsweise einer Betätigungsstange umgesetzt wird.

Bei aus dem Stand der Technik bekannten Betätigungseinrichtungen sind insbesondere in einem für eine Klemmkraftbereitstellung erforderlichen, hohen Kraftbereich lange Betätigungswege zum Aufbringen einer Betätigungskraft erforderlich. Lange Betätigungswege führen mitunter zu einer weiten Betätigungshebelstellung und einer Verminderung der Bedienbarkeit solcher Instrumente.

Der Erfindung liegt die Aufgabe zugrunde, die Bedienbarkeit der Betätigungseinrichtung und damit die Bedienbarkeit eines Instruments mit einer solchen Betätigungseinrichtung zu verbessern.

Diese Aufgabe wird gelöst durch eine Betätigungseinrichtung mit den Merkmalen des Anspruchs 1 und durch ein Instrument mit den Merkmalen des Anspruchs 13.

Eine Ausführungsform betrifft eine Betätigungseinrichtung für ein medizinisches Instrument. Die Betätigungseinrichtung umfasst einen Grundkörper mit einem an dem Grundkörper angeordneten Griffelement und einen Betätigungshebel. Über ein Betätigen des Betätigungshebels an einem proximalen Ende des Instruments von distal aus einer ersten Stellung, insbesondere einer Ruhestellung, nach proximal in eine zweite Stellung, insbesondere eine Arbeitsstellung, und/oder von proximal aus der zweiten Stellung nach distal in die erste Stellung sind an einem distalen Ende des Instruments vorgesehene Werkzeugelemente bewegbar. Der Betätigungshebel ist relativ zu dem Griffelement der Betätigungseinrichtung rotatorisch bewegbar und die Betätigungseinrichtung ist derart ausgebildet, dass eine rotatorische Eingangsbewegung des Betätigungshebels durch ein entlang einer Schaftachse des Grundkörpers translatorisch verschiebbares Übertragungsglied in eine translatorische Ausgangsbewegung zum Bewegen der Werkzeugelemente umgesetzt wird.

Zwischen dem Betätigungshebel und dem Übertragungsglied ist ein entsprechend ausgebildeter Kipphebel angeordnet, der sowohl mit dem Betätigungshebel als auch mit dem Übertragungsglied drehbar, beispielsweise über je ein Drehgelenk, verbunden ist. Das Übertragungsglied ist beispielsweise in einer Führung der Betätigungseinrichtung angeordnet und wird dementsprechend translatorisch geführt.

Eine Drehachse, um die der Betätigungshebel rotatorisch bewegbar ist, translatorisch in Bezug auf den Grundkörper gelagert, derart, dass durch die rotarische Eingangsbewegung des Betätigungshebels die Drehachse translatorisch verschiebbar ist. Die translatorische Verschiebung der Rotationsachse ist bevorzugt eine geradlinig translatorische Verschiebung. Die Rotationsachse wird demnach entlang einer Geraden verschoben.

Gemäß einer Ausführungsform ist vorgesehen, dass die Drehachse durch die rotarische Eingangsbewegung des Betätigungshebels translatorisch parallel zu der Schaftachse des Grundkörpers 18 verschiebbar ist. Es ist auch denkbar, dass die Drehachse vertikal oder schräg zur Schaftachse des Grundkörpers verschiebbar ist.

Gemäß einer Ausführungsform ist vorgesehen, dass ein Lagerungsglied, das die Drehachse des Betätigungshebels umfasst, in einer Führung des Grundkörpers, insbesondere in einer Führungsnut eines Gehäuses des Grundkörpers, gelagert ist. Das Lagerungsglied ist beispielsweise in einer in einem Gehäuse des Grundkörpers ausgebildeten Führungsnut rotatorisch und translatorisch gelagert. Beispielsweise verläuft die Führung, insbesondere die Führungsnut, parallel zur Schaftachse. Das Lagerungsglied und damit die Drehachse sind demnach in der Führung rotatorisch und translatorisch entlang der Schaftachse verschiebbar.

Vorteilhafterweise umfasst der Betätigungshebel, insbesondere das Lagerungsglied des Betätigungshebels, eine distale Führungskurve und eine proximale Führungskurve, wobei die Führungskurven durch Betätigen des Betätigungshebels entlang wenigstens eines am Grundkörper angeordneten Stößels abgleiten.

Gemäß einer Ausführungsform sind die Führungskurven durch eine, insbesondere gekrümmte, Führungsnut gebildet und der Stößel ist in der Führungsnut angeordnet. Die Breite der Führungsnut ist derart auf den Durchmesser des Stößels abgestimmt, dass der Stößel zwischen der distalen Führungskurve und der proximalen Führungskurve mit Spiel geführt ist und die beiden Führungskurven beim Betätigen des Betätigungshebels am Stößel abgleiten.

Gemäß einer Ausführungsform erfolgt das translatorische Verschieben der Drehachse in Abhängigkeit einer Form der, insbesondere gekrümmten, Führungsnut. Über eine Ausbildung der Form der Führungsnut wird somit das translatorische Verschieben der Drehachse vorgegeben.

In Weiterbildung dieses Erfindungsgedankens ist vorgesehen, dass der Stößel die Führungsnut in einem jeweiligen Kontaktpunkt kontaktiert und eine Form der Führungsnut in Bezug auf die Drehachse über den Verlauf der Führungsnut derart ausgebildet ist, dass sich ein Abstand eines jeweiligen Kontaktpunkts zur Drehachse ausgehend von der ersten Stellung der Betätigungseinrichtung zur zweiten Stellung vergrößert.

Vorteilhafterweise wird durch Zusammenwirken von Stößel und Führungsnut ein distales Gegenlager und ein proximales Gegenlager bereitgestellt, so dass die Drehachse in jeder Stellung gegen ein selbsttätiges translatorisches Verschieben ohne Betätigen des Betätigungshebels gesichert ist.

Alternativ wäre auch eine Ausführungsform denkbar, bei der sich die Führungsnut nicht am Lagerungsglied des Betätigungshebels, sondern in Form von zwei spiegelsymmetrisch in einem Gehäuse der Betätigungseinrichtung ausgebildeten, lateral gegenüberliegenden und einander zugewandten Führungsnuten erstreckt. Der Stößel ist in diesem Fall beidseitig an dem Lagerungsglied des Betätigungshebels angeordnet und gleitet während der Betätigung entlang der Führungsnuten in dem Gehäuse der Betätigungseinrichtung. Auch auf diese Weise wäre eine Verschiebung der Drehachse im Verlauf der Betätigung umsetzbar.

Gemäß einer weiteren Ausführungsform ist vorgesehen, dass der Grundkörper einen distalen und einen proximalen Stößel umfasst, und wobei der distale Stößel des Grundkörpers distal in Bezug auf das Lagerungsglied am Grundkörper angeordnet ist, und der proximale Stößel proximal in Bezug auf das Lagerungsglied am Grundkörper angeordnet ist.

In diesem Fall ist vorgesehen, dass die distale Führungskurve an dem distalen Stößel abgleitet und die proximale Führungskurve an dem proximalen Stößel abgleitet. Beim Betätigen des Betätigungshebels aus der ersten Stellung, beispielsweise der Ruhestellung, in die zweite Stellung, beispielsweise die Arbeitsstellung, wirkt der distale Stößel als Gegenlager, wodurch die Rotationsachse nach proximal verschoben wird. Beim Betätigen des Betätigungshebels aus der zweiten Stellung in die erste Stellung wirkt der proximale Stößel entsprechend als Gegenlager, wodurch die Drehachse wieder nach distal verschoben wird.

In Weiterbildung dieses Erfindungsgedanken erweist es sich als vorteilhaft, dass der distale Stößel die distale Führungskurve in einem jeweiligen Kontaktpunkt kontaktiert und der proximale Stößel die proximale Führungskurve in einem jeweiligen Kontaktpunkt kontaktiert, und die distale Führungskurve derart ausgebildet ist, dass sich ein Abstand des Kontaktpunkts zur Drehachse ausgehend von der ersten Stellung der Betätigungseinrichtung zur zweiten Stellung vergrößert, und die proximale Führungskurve derart ausgebildet ist, dass sich ein Abstand des Kontaktpunkts zur Drehachse ausgehend von der ersten Stellung der Betätigungseinrichtung zur zweiten Stellung verkleinert. Die Abstände der Kontaktpunkte des distalen Stößels und der distalen Führungskurve und des proximalen Stößels und der proximalen Führungskurve vergrößern bzw. verkleinern sich demnach entsprechend gegenläufig.

Vorteilhafterweise ist die Ausbildung der distalen und der proximalen Führungskurve derart aufeinander abstimmt, dass durch Zusammenwirken des distalen Stößels mit der distalen Führungskurve ein distales Gegenlager und durch Zusammenwirken des proximalen Stößels mit der proximalen Führungskurve ein proximales Gegenlager bereitgestellt wird, so dass die Drehachse in jeder Stellung gegen ein selbsttätiges translatorisches Verschieben ohne Betätigen des Betätigungshebels gesichert ist. Beispielsweise ist der effektive Durchmesser des Lagerungsglieds im Bereich der Führungskurven konstant.

Die proximale und die distale Führungskurve sind so aufeinander abgestimmt, dass ein Bewegen des Betätigungshebels ermöglicht wird. Das Lagerungsglied ist also nicht zwischen dem proximalen und dem distalen Stößel eingeklemmt. Andererseits sind die proximale und die distale Führungskurve so aufeinander abgestimmt, dass der translatorische Freiheitsgrad der Rotationsachse in jeder Hebelstellung des Betätigungshebels durch die beiden Stößel gesperrt ist und nur durch Betätigen des Betätigungshebels selbst ein geführtes translatorisches Verschieben ermöglicht wird.

Es kann vorgesehen sein, dass die Betätigungseinrichtung ein Rückstellelement umfasst, zum Rückstellen des Betätigungshebels aus der zweiten Stellung, insbesondere der Arbeitsstellung, in die erste Stellung, insbesondere die Ruhestellung.

Weitere Ausführungsformen betreffen ein medizinisches Instrument, wobei das Instrument eine Betätigungseinrichtung nach einem der vorstehenden Ansprüche umfasst, wobei die Betätigungseinrichtung mit an einem distalen Ende des Instruments angeordneten Werkzeugelementen zusammenwirkend an einem proximalen Ende des Instruments angeordnet oder ausgebildet ist, und wobei die Betätigungseinrichtung zum Bewegen der Werkzeugelemente relativ zueinander ausgebildet ist.

Gemäß einer Ausführungsform ist das medizinische Instrument in Form eines elektrochirurgischen Instruments und/oder in Form eines Klammergeräts ausgebildet und/oder umfasst ein elektrochirurgisches Instrument und/oder ein Klammergerät.

Weitere Vorteile ergeben sich aus der Beschreibung und den beigefügten Zeichnungen. Ausführungsbeispiele der Erfindung sind in den Zeichnungen dargestellt und werden in der nachfolgenden Beschreibung näher erläutert. Dabei bezeichnen gleiche Bezugszeichen in verschiedenen Figuren jeweils gleiche oder zumindest ihrer Funktion nach vergleichbare Elemente. Bei der Beschreibung einzelner Figuren wird gegebenenfalls auch auf Elemente aus anderen Figuren Bezug genommen. Es zeigen jeweils in schematischer Form:
- Fig. 1: eine aus dem Stand der Technik bekannte Betätigungseinrichtung für ein medizinisches Instrument;
- Fig. 2: charakteristische Betätigungsverläufe verschiedener Betätigungseinrichtungen;
- Fig. 3a): einen Ausschnitt einer erfindungsgemäßen Betätigungseinrichtung gemäß einer ersten Ausführungsform in einer ersten Stellung;
- Fig. 3b): einen Ausschnitt der erfindungsgemäßen Betätigungseinrichtung gemäß der ersten Ausführungsform in einem Übergang von der ersten Stellung in eine zweite Stellung;
- Fig. 3c): einen Ausschnitt der erfindungsgemäßen Betätigungseinrichtung gemäß der ersten Ausführungsform in der zweiten Stellung;
- Fig. 4: eine erfindungsgemäße Betätigungseinrichtung gemäß einer weiteren Ausführungsform in einer ersten Stellung.

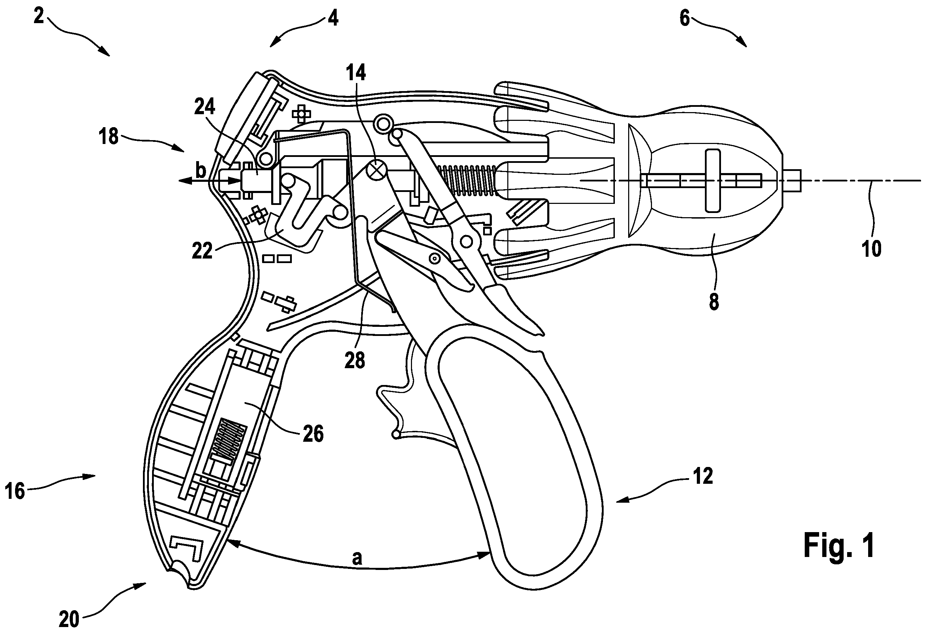

In Fig. 1 ist ein Ausführungsbeispiel einer aus dem Stand der Technik bekannten Betätigungseinrichtung 2 für ein medizinisches Instrument schematisch dargestellt. Das medizinische Instrument ist beispielsweise in Form eines elektrochirurgischen Instruments (nicht weiter dargestellt) ausgebildet. Vorliegend handelt es sich um ein sogenanntes Seal & Cut Instrument, bei dem mittels des Betätigungshebels 12 das Instrumentenmaul geschlossen und geöffnet werden kann. Im geschlossenen Zustand des Instrumentenmauls kann eine Versiegelung ("Seal") eines Gewebes (zumeist ein Hohlorgan oder Blutgefäß) mittels elektronischer Koagulation (HF Koagulation) hergestellt werden. Im geschlossenen Zustand des Instrumentenmauls kann zudem mittels Betätigung eines weiteren Betätigungselements eine Schneidklinge durch das geschlossene Instrumentenmaul vorgeschoben werden ("Cut"), um das gegriffene Gewebe nach der Koagulation zu trennen. Alternativ dazu ist aber auch ein elektronisches Schneiden mittels HF Strom und/oder ein Ultraschallschneiden möglich. Ein solches Instrument weist ein proximales Ende 4 und ein distales Ende 6 auf.

Die in Fig. 1 dargestellte Betätigungseinrichtung 2 ist beispielsweise an dem proximalen Ende 4 des Instruments angeordnet. Die Betätigungseinrichtung 2 ist beispielsweise relativ zu einem langgestreckten Schaft 8 des Instruments um eine von diesem definierte Schaftachse 10 verdrehbar angeordnet.

An der Betätigungseinrichtung 2 steht in etwa quer zur Schaftachse 10 ein Betätigungshebel 12 ab, welcher in proximaler Richtung (und distaler Richtung) um eine quer zur Schaftachse 10 verlaufende Schwenkachse 14 in Richtung auf ein feststehendes Griffelement 16 verschwenkbar ist. Das Griffelement 16 steht ebenfalls in etwa quer bezogen auf die Schaftachse 10 von einem Grundkörper 18 der Betätigungseinrichtung 2 ab. Der Grundkörper 18 bildet oder umfasst, insbesondere zusammen mit dem Griffelement 16 und dem Schaft 8, ein Gehäuse der Betätigungseinrichtung 2. Ein solches Gehäuse umfasst beispielsweise zwei schalenförmige Gehäuseteile, die in einem montierten Zustand Teile des Betätigungsmechanismus der Betätigungseinrichtung 40 umschließen. In den Figuren ist das Gehäuse in einem geöffneten Zustand, nämlich mit nur einem schalenförmigen Gehäuseteil, dargestellt.

An einem freien Ende 20 des Griffelements 16 ist beispielsweise ein nicht weiter dargestelltes Anschlusskabel zur Verbindung mit einer Stromversorgungseinrichtung herausführbar.

An dem distalen Ende 6 des Instruments sind beispielsweise nicht weiter dargestellte Werkzeugelemente, beispielsweise ein erstes Werkzeugelement und ein zweites Werkzeugelement, relativ zueinander bewegbar, nämlich verschwenkbar, angeordnet.

Das erste Werkzeugelement ist über ein nicht weiter dargestelltes Kraftübertragungsglied (beispielsweise einen Zugdraht) mit dem Betätigungshebel 12 gekoppelt, sodass infolge einer Verschwenkbewegung des Betätigungshebel 12 in Richtung auf das Griffelement 16 hin das erste Werkzeugelement in Richtung auf das zweite Werkzeugelement hin verschwenkbar ist.

In Fig. 1 ist die Betätigungseinrichtung 2 in einer ersten Stellung, beispielsweise einer Ruhestellung, dargestellt. In dieser Stellung sind die an dem distalen Ende 6 des Instruments angeordneten Werkzeugelemente, auch Instrumentenmaul, geöffnet.

Die Betätigungseinrichtung 2 kann durch Betätigen des Betätigungshebels 12 von distal aus der Ruhestellung nach proximal in eine zweite Stellung, beispielsweise eine Arbeitsstellung, gebracht werden. Dabei wird eine rotatorische Eingangsbewegung um die Schwenkachse 14, vgl. Doppelpfeil a, des Betätigungshebels auf einen Kipphebel 22 übertragen. Der Kipphebel 22 ist zum einen an dem Betätigungshebel 12 durch ein Drehgelenk gelagert und zum anderen an einem Übertragungsglied 24 ebenfalls durch ein Drehgelenk gelagert. Das Übertragungsglied 24 ist translatorisch verschiebbar in einer entsprechenden Führungsnut des Grundkörpers 18 angeordnet. Der Kipphebel führt demnach eine Bewegung aus, die einen rotarischen und einen translatorischen Anteil umfasst. Über den Kipphebel 22 wird die rotatorische Eingangsbewegung a in eine translatorische Bewegung, vgl. Doppelpfeil b, des Übertragungsglieds 24 übertragen, in dem das Übertragungsglied 24 den translatorischen Anteil der Bewegung des Kipphebels 22 aufnimmt. Das Übertragungsglied überträgt die translatorische Bewegung b über ein nicht weiter dargestelltes Kraftübertragungsglied (beispielsweise einen Zugdraht) auf ebenfalls nicht weiter dargestellte Werkzeugelemente. In der Arbeitsstellung sind die an dem distalen Ende 6 des Instruments angeordneten Werkzeugelemente, auch als Instrumentenmaul bezeichnet, geschlossen.

Abhängig von einer Kraft- und Wegrichtung der Eingangsbewegung a führt die Betätigung des Betätigungshebels 12 beispielsweise zu einem Öffnen oder Schließen der Werkzeugelemente, bei denen es sich beispielsweise um zur Gefäßversiegelung verwendete Maulteile handelt.

Gemäß dem dargestellten Beispiel erreicht der Betätigungshebel 12 in der Arbeitsstellung bei vollständiger Betätigung des Betätigungshebels 12 in proximaler Richtung eine Umlaufsperre 26. Beim Erreichen der Arbeitsstellung ist zwischen den Werkzeugelementen, insbesondere den Maulteilen, die für die Gefäßversiegelung notwendige, mechanische Klemmkraft zwischen den Maulteilen aufgebaut. Die Umlaufsperre 26 sperrt den Betätigungshebel 12 in der Arbeitsstellung bis der Betätigungshebel 12 manuell aus der Umlaufsperre gelöst wird. Ein Rückstellelement 28, beispielsweise eine Rückstellfeder, wirkt auf den Betätigungshebel 12, so dass wieder in distaler Richtung zurück in die in Fig. 1 gezeigte Ruhestellung bewegt wird, und sich die Werkzeugelemente wieder vollständig öffnen.

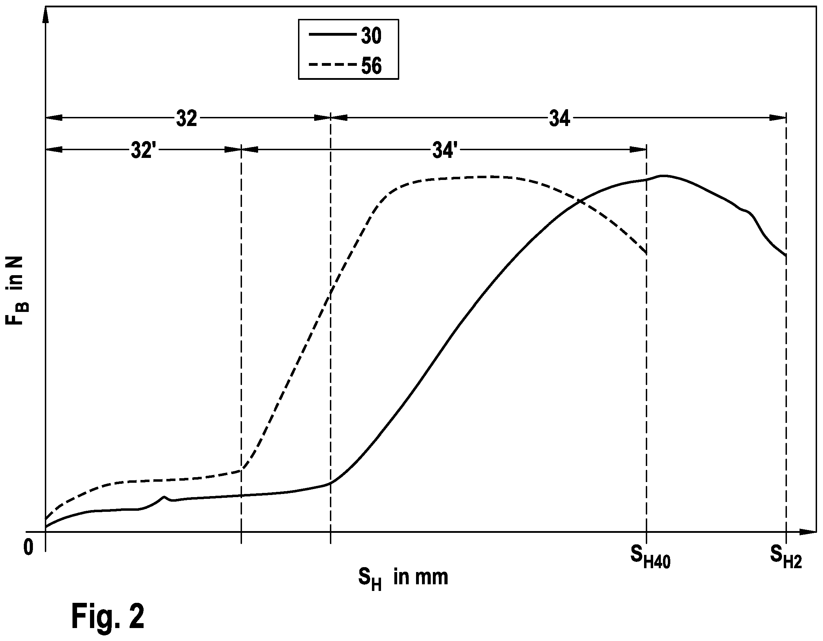

In Fig. 2 ist beispielhaft ein Verlauf einer an dem Betätigungshebel 12 der aus dem Stand der Technik bekannten Betätigungseinrichtung 2 wirkenden Betätigungskraft F_{B}, vgl. durchgezogene Linie, in Abhängigkeit eines Betätigungswegs S_{H} des Betätigungshebel 12 dargestellt. Der Betätigungsweg ist im Beispiel ausgehend von der ersten Stellung, im Beispiel die Ruhestellung, im Achsenschnittpunkt (0,0), bis in eine zweite Stellung, im Beispiel die Arbeitsstellung, dargestellt. Für die Betätigungseinrichtung 2 ist die Arbeitsstellung bei S_{H2}.

Bei einem aus dem Stand der Technik bekannten Betätigungshebel 12 ergibt sich, insbesondere bei wenig beladenem Maulteil, die mit einer durchgezogenen Linie dargestellte charakteristische Betätigungskraftkurve 30. Die Betätigungskraftkurve 30 kann in zwei Abschnitte, Abschnitt 32 und Abschnitt 34 aufgeteilt werden. Der erste Abschnitt 32 umfasst die Maulteilöffnung bzw. -schließung in einem verhältnismäßig niedrigen Kraftbereich. Der zweite Abschnitt 34 umfasst den Klemmkraftaufbau bis zum Erreichen der Arbeitsstellung in einem verhältnismäßig hohen Kraftbereich.

Im zweiten Abschnitt 34 ist dem Übersetzungsverlauf geschuldet ein niedriger Kraftanstieg der Betätigungskraft hin zu einer Maximalkraft zu beobachten. Danach kann ein Abfallen der Betätigungskraft bis zum Erreichen der Arbeitsstellung bei S_{H2} beobachtet werden. Dies resultiert in einem insgesamt langen Betätigungsweg, insbesondere für den zweiten Abschnitt 34 im hohen Kraftbereich. Dieser führt wiederum zu einer weiten Betätigungshebelstellung in der Ruhestellung und einer Verminderung der Bedienbarkeit der Betätigungseinrichtung 2. Im Folgenden werden unter Bezugnahme auf die Figuren 2 bis 5 verschiedene Ausführungsformen einer erfindungsgemäßen Betätigungseinrichtung 40 erläutert.

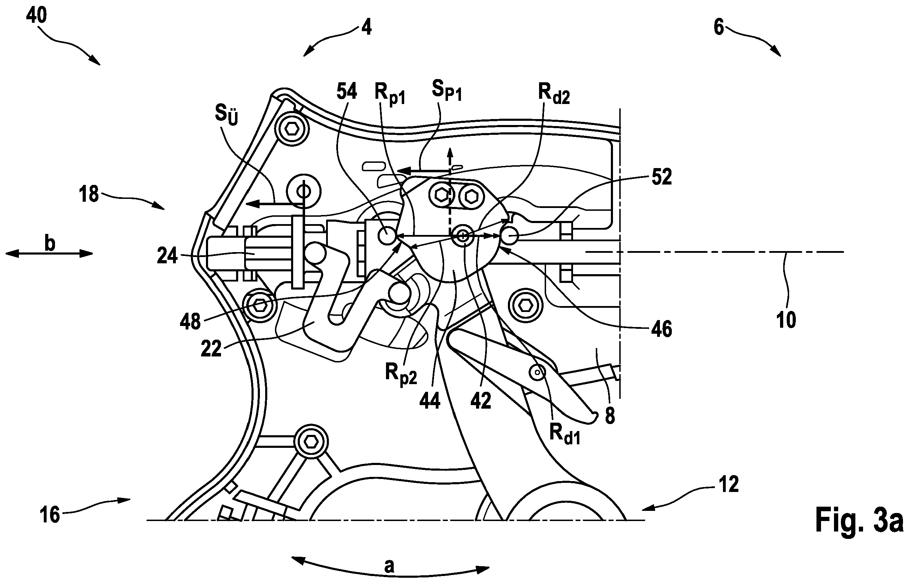

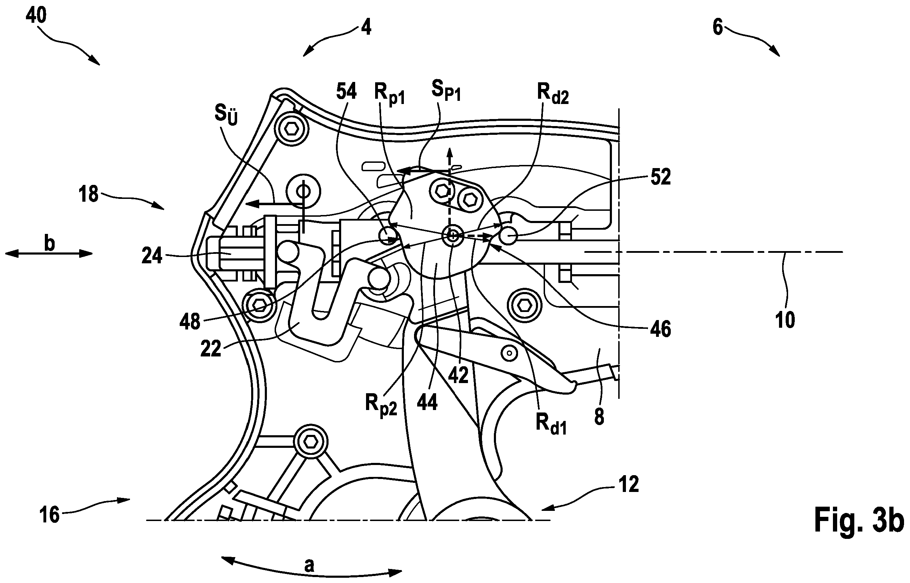

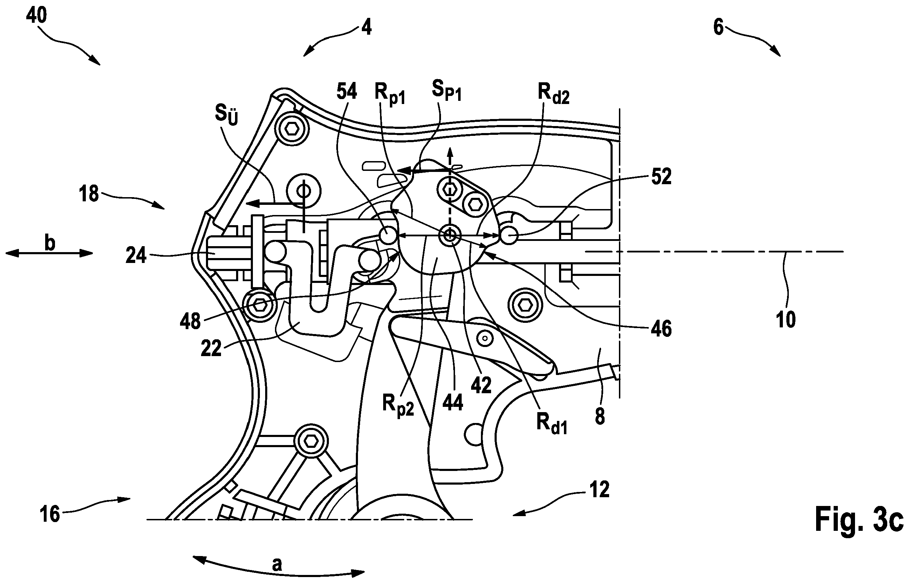

Die Figuren 3a) bis 3c) zeigen einen Ausschnitt einer Betätigungseinrichtung 40 gemäß einer ersten Ausführungsform in einer ersten Stellung, im Beispiel eine Ruhestellung (Fig. 3a)), in einem Übergang von der Ruhestellung in eine zweite Stellung, im Beispiel eine Arbeitsstellung, ab Beginn eines Klemmkraftaufbaus (Fig. 3b)), und in der Arbeitsstellung (Fig. 3c)).

Bei der Betätigungseinrichtung 40 ist vorgesehen, dass der Betätigungshebel 12 um eine Drehachse 42 rotatorisch bewegbar ist. Die Drehachse 42 ist gemäß dem Beispiel translatorisch in Bezug auf den Grundkörper 18 gelagert. Durch die rotarische Eingangsbewegung a des Betätigungshebels 12 ist die Drehachse 42 translatorisch verschiebbar. Gemäß der dargestellten Ausführungsform ist die translatorische Verschiebung der Rotationsachse eine geradlinig translatorische Verschiebung. Die Rotationsachse wird demnach entlang einer Geraden verschoben.

Gemäß der dargestellten Ausführungsform ist die Drehachse 42 durch die rotarische Eingangsbewegung a des Betätigungshebels 12 translatorisch parallel zu der Schaftachse 10 des Grundkörpers 18 verschiebbar.

Der Betätigungshebel 12 umfasst ein Lagerungsglied 44. Das Lagerungsglied 44 umfasst die Drehachse 42 des Betätigungshebels 12. Das Lagerungsglied ist beispielsweise in einer Führung des Grundkörpers 18, insbesondere in einer in einem Gehäuse des Grundkörpers 18 ausgebildeten Führungsnut, rotatorisch und translatorisch gelagert. Gemäß dem Beispiel verläuft die Führung parallel zur Schaftachse 10. Das Lagerungsglied 44 und damit die Drehachse 42 sind demnach in der Führung rotatorisch und translatorisch entlang der Schaftachse 10 verschiebbar. Im Beispiel wird während der Betätigung aus der Ruhestellung in die Arbeitsstellung die Drehachse 42 um einen Gesamtweg von etwa 1mm bis 3mm, insbesondere 2mm, parallel zu der Schaftachse 10 verschoben.

Im Beispiel umfasst der Betätigungshebel 12 an dem Lagerungsglied 44 eine distale Führungskurve 46 und eine proximale Führungskurve 48. Beim Betätigen des Betätigungshebels 12 gleiten die Führungskurven 46, 48 jeweils an einem am Grundkörper angeordneten Stößel 50 ab. Im Beispiel gleitet distale Führungskurve 46 an einem distalen Stößel 52 und die proximale Führungskurve 48 an einem proximalen Stößel 54 ab.

Das translatorische Verschieben der Drehachse 42 erfolgt also durch Betätigen des Betätigungshebels, wobei beim Betätigen des Betätigungshebels von distal aus der Ruhestellung nach proximal in die Arbeitsstellung der distale Stößel eine Art Gegenlager bildet und dadurch die Drehachse 42 translatorisch nach proximal verschoben wird, und beim Betätigen des Betätigungshebels von proximal aus der Arbeitsstellung nach distal in die Ruhestellung der proximale Stößel eine Art Gegenlager bildet und dadurch die Drehachse 42 translatorisch nach distal verschoben wird. Wie vorstehend erläutert, können dadurch Werkzeugelemente geöffnet und geschlossen bzw. eine Klemmkraft auf- und abgebaut werden.

Im Beispiel kontaktiert der distale Stößel 52 die distale Führungskurve 46 in einem jeweiligen Kontaktpunkt und der proximale Stößel 54 kontaktiert die proximale Führungskurve 48 in einem jeweiligen Kontaktpunkt. Die distale Führungskurve 46 ist derart ausgebildet, dass sich ein Abstand des Kontaktpunkts zur Drehachse 42 ausgehend von der ersten Stellung der Betätigungseinrichtung 40, vgl. Pfeil R_{d1}, zur zweiten Stellung, vgl. Pfeil R_{d2}, vergrößert. Die proximale Führungskurve 48 ist derart ausgebildet, dass sich ein Abstand des Kontaktpunkts zur Drehachse 42 ausgehend von der ersten Stellung der Betätigungseinrichtung 40, vgl. Pfeil Rₚ₁, zur zweiten Stellung, vgl. Pfeil Rₚ₂, verkleinert.

Die Ausbildung der proximalen und der distalen Führungskurve 46, 48 sind im Beispiel derart aufeinander abstimmt, dass durch Zusammenwirken des distalen Stößels 52 mit der distalen Führungskurve 46 ein distales Gegenlager und durch Zusammenwirken des proximalen Stößels 54 mit der proximalen Führungskurve 48 ein proximales Gegenlager bereitgestellt wird, so dass die Drehachse 42 in jeder Stellung gegen ein selbsttätiges translatorisches Verschieben ohne Betätigen des Betätigungshebels gesichert ist.

Das translatorische Verschieben der Drehachse 42 gemäß der in den Figuren 3a) bis 3c) dargestellten Ausführungsform ermöglicht die Umsetzung eines schnelleren Kraftanstieges auf die Maximalkraft, vgl. die in Fig. 2 als gestrichelte Linie dargestellte charakteristische Betätigungskraftkurve 56.

Die Betätigungskraftkurve 56 kann in zwei Abschnitte, Abschnitt 32' und Abschnitt 34' aufgeteilt werden. Der erste Abschnitt 32' umfasst ausgehend von der Ruhestellung der Betätigungseinrichtung 40 in einem verhältnismäßig niedrigen Kraftbereich die Maulteilöffnung bzw. -schließung beim Übergang von Abschnitt 32' zu Abschnitt 34'. Der zweite Abschnitt 34' umfasst den Klemmkraftaufbau bis zum Erreichen der Arbeitsstellung der Betätigungseinrichtung 40 in einem verhältnismäßig hohen Kraftbereich.

Gemäß den dargestellten Betätigungskurven 30, 56 liegt die wirkende Betätigungskraft F_{B} im Bereich 32, 32' bei der erfindungsgemäßen Betätigungseinrichtung 40 über der Betätigungskraft F_{B} der aus dem Stand der Technik bekannten Betätigungseinrichtung 2. Bei der erfindungsgemäßen Betätigungseinrichtung 40 ist der Betätigungsweg S_{H} des Betätigungshebels 12 im Bereich 32, 32' kürzer als bei der aus dem Stand der Technik bekannten Betätigungseinrichtung 2. Bei der erfindungsgemäßen Betätigungseinrichtung 40 ist dementsprechend ein kürzerer Betätigungsweg S_{H} des Betätigungshebels 12 erforderlich um den Bereich des Klemmkraftaufbaus, vgl. Bereich 34', zu erreichen. Bei etwa gleicher Fläche der Betätigungskraftkurven 30, 56 im Bereich 32' und Bereich 32 und damit der etwa gleichen verrichteten Arbeit wird somit insgesamt weniger Betätigungsweg S_{H} des Betätigungshebels 12 benötigt.

Erfindungsgemäß wird dies durch die Verschiebung der Position S_{P1} der Drehachse 42 erreicht. Bei der erfindungsgemäßen Betätigungseinrichtung 40 ändert sich sowohl die Position S_{P1} der Drehachse 42 aufgrund der translatorischen Verschiebung über den gesamten Betätigungsweg als auch die Position S_{Ü} des Übertragungsglieds, vgl. auch die Pfeile S_{P1} und S_{Ü} in Fig. 3a bis 3c. Im Vergleich dazu verändert sich eine Position der Schwenkachse 14 der aus dem Stand der Technik bekannten Betätigungsrichtung 2 nicht.

Die Erfindung ermöglicht somit in Abhängigkeit von der Verschiebung bzw. Position der Drehachse 42 und den Radien der Führungskurven 46, 48 am Lagerungsglied 44 eine Reduzierung des Gesamtbetätigungsweges bei annähernd gleichbleibender Maximalkraft. Daraus resultiert ein geringerer Öffnungswinkel des Betätigungshebels in der Ruhestellung, was sich allgemein positiv auf die Handhabung des Instrumentes auswirkt.

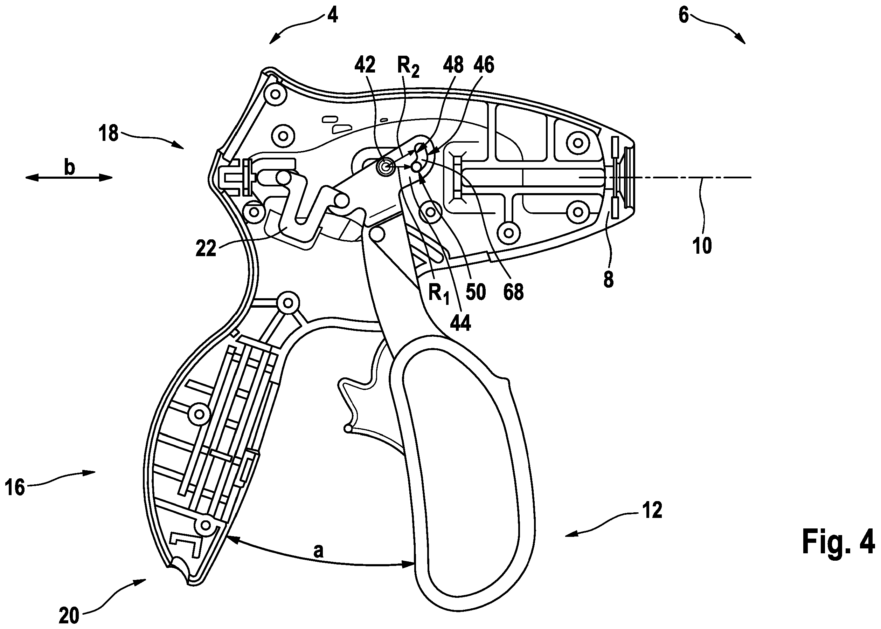

Fig. 4 zeigt schließlich eine weitere Ausführungsform der erfindungsgemäßen Betätigungseinrichtung 40. Gemäß dieser Ausführungsform sind die Führungskurven 46, 48 durch eine gekrümmte Führungsnut 68 gebildet. Der Stößel 50 ist in der Führungsnut angeordnet. Die distale Führungskurve 46 und die proximale Führungskurve 48 werden demnach durch die distale und proximale Begrenzung der Führungsnut 68 bereitgestellt.

Eine Breite der Führungsnut 68 ist derart auf den Durchmesser des Stößels 50 abgestimmt, dass der Stößel 50 zwischen der distalen Führungskurve 46 und der proximalen Führungskurve 48 beidseits mit Spiel geführt wird, und die beiden Führungskurven 46, 48 beim Betätigen des Betätigungshebels am Stößel abgleiten.

Das translatorische Verschieben der Drehachse 42 erfolgt in Abhängigkeit einer Form der gekrümmten Führungsnut 68. Der Stößel 50 kontaktiert die Führungsnut in einem jeweiligen Kontaktpunkt. Im Beispiel ist die Form der Führungsnut 68 in Bezug auf die Drehachse 42 über den Verlauf der Führungsnut 68 derart ausgebildet ist, dass sich ein Abstand eines jeweiligen Kontaktpunkts zur Drehachse ausgehend von der ersten Stellung der Betätigungseinrichtung 40, vgl. Pfeil R₁, zur zweiten Stellung, vgl. Pfeil R₂, vergrößert.

Durch Zusammenwirken von Stößel 50 und Führungsnut 68 wird ein distales Gegenlager und ein proximales Gegenlager bereitgestellt, so dass die Drehachse 42 in jeder Stellung gegen ein selbsttätiges translatorisches Verschieben ohne Betätigen des Betätigungshebels 12 gesichert ist.

In Fig. 4 sind einige Elemente, beispielsweise das Übertragungsglied 24 und weitere Elemente, nicht dargestellt. Diese können bei der in Fig. 4 dargestellten Ausführungsform analog zu den in den Fig. 1 und Fig. 3 dargestellten Ausführungsformen vorgesehen werden. In den Fig. 3 und 4 ist kein Rückstellelement 28 und keine Umlaufsperre 26, vgl. Fig. 1, dargestellt. Diese Elemente können bei den in den Fig. 3 und 4 dargestellten Ausführungsformen gemäß der Darstellung in Fig. 1 ebenfalls vorgesehen werden.

### Bezugszeichenliste

- 2: Betätigungseinrichtung
- 4: proximales Ende
- 6: distales Ende
- 8: Schaft
- 10: Schaftachse
- 12: Betätigungshebel
- 14: Schwenkachse
- 16: Griffelement
- 18: Grundkörper
- 20: freies Ende (Griffelement)
- 22: Kipphebel
- 24: Übertragungsglied
- 26: Umlaufsperre
- 28: Rückstellelement
- 30: Betätigungskraftkurve (Stand der Technik)
- 32: Abschnitt 1
- 34: Abschnitt 2

- 40: Betätigungseinrichtung
- 42: Drehachse
- 44: Lagerungsglied
- 46: distale Führungskurve
- 48: proximale Führungskurve
- 50: Stößel
- 52: distaler Stößel
- 54: proximaler Stößel
- 56: Betätigungskraftkurve (erfindungsgemäß)
- 58: Verlauf
- 60: Verlauf
- 62: Bereich
- 64: Bereich
- 66: Bereich
- 68: Führungsnut

## Patentansprüche

1. Betätigungseinrichtung (40) für ein medizinisches Instrument, umfassend einen Grundkörper (18) mit einem an dem Grundkörper (18) angeordneten Griffelement (16) und einen Betätigungshebel (12), wobei über ein Betätigen des Betätigungshebels (12) an einem proximalen Ende (4) des Instruments von distal aus einer ersten Stellung, insbesondere einer Ruhestellung, nach proximal in eine zweite Stellung, insbesondere eine Arbeitsstellung, und/oder von proximal aus der zweiten Stellung nach distal in die erste Stellung an einem distalen Ende (6) des Instruments vorgesehene Werkzeugelemente bewegbar sind, wobei der Betätigungshebel (12) relativ zu dem Griffelement (16) der Betätigungseinrichtung (40) rotatorisch bewegbar ist, und die Betätigungseinrichtung (40) derart ausgebildet ist, dass eine rotatorische Eingangsbewegung (a) des Betätigungshebels (12) mittels einem entlang einer Schaftachse (10) des Grundkörpers (18) translatorisch verschiebbaren Übertragungsglied (24) in eine translatorische Ausgangsbewegung (b) zum Bewegen der Werkzeugelemente umgesetzt wird, wobei zwischen dem Betätigungshebel (12) und dem Übertragungsglied (24) ein Kipphebel (22) angeordnet ist, wobei der Kipphebel (22) drehbar mit dem Betätigungshebel (12) und drehbar mit dem Übertragungsglied (24) verbunden ist, und wobei eine Drehachse (42), um die der Betätigungshebel (12) rotatorisch bewegbar ist, translatorisch in Bezug auf den Grundkörper (18) gelagert ist, derart, dass durch die rotarische Eingangsbewegung (a) des Betätigungshebels (12) die Drehachse (42) translatorisch verschiebbar ist.

2. Betätigungseinrichtung (40) nach Anspruch 1, wobei durch die rotarische Eingangsbewegung (a) des Betätigungshebels (12) die Drehachse (42) translatorisch parallel zu der Schaftachse (10) des Grundkörpers (18) verschiebbar ist.

3. Betätigungseinrichtung (40) nach einem der Ansprüche 1 oder 2, wobei ein Lagerungsglied (44), das die Drehachse (42) des Betätigungshebels (12) umfasst, in einer Führung des Grundkörpers (18), insbesondere in einer Führungsnut eines Gehäuses des Grundkörpers (18), gelagert ist.

4. Betätigungseinrichtung (40) nach einem der vorhergehenden Ansprüche, wobei der Betätigungshebel (12), insbesondere das Lagerungsglied (44) des Betätigungshebels (12), eine distale Führungskurve (46) und eine proximale Führungskurve (48) umfasst, und die Führungskurven (46, 48) durch Betätigen des Betätigungshebels (12) entlang wenigstens eines am Grundkörper (18) angeordneten Stößels (50) abgleiten.

5. Betätigungseinrichtung (40) nach Anspruch 4, wobei die Führungskurven (46, 48) durch eine, insbesondere gekrümmte, Führungsnut (68) gebildet sind, und der Stößel (50) in der Führungsnut (68) angeordnet ist.

6. Betätigungseinrichtung (40) nach Anspruch 5, wobei das translatorische Verschieben der Drehachse (42) in Abhängigkeit einer Form der, insbesondere gekrümmten, Führungsnut (68) erfolgt.

7. Betätigungseinrichtung (40) nach einem der Ansprüche 5 oder 6, wobei der Stößel die Führungsnut in einem jeweiligen Kontaktpunkt kontaktiert und eine Form der Führungsnut (68) in Bezug auf die Drehachse (42) über den Verlauf der Führungsnut (68) derart ausgebildet ist, dass sich ein Abstand eines jeweiligen Kontaktpunkts zur Drehachse ausgehend von der ersten Stellung der Betätigungseinrichtung (40) zur zweiten Stellung vergrößert.

8. Betätigungseinrichtung (40) nach einem der Ansprüche 5 bis 7, wobei durch Zusammenwirken von Stößel (50) und Führungsnut (68) ein distales Gegenlager und ein proximales Gegenlager bereitgestellt wird, so dass die Drehachse (42) in jeder Stellung gegen ein selbsttätiges translatorisches Verschieben ohne Betätigen des Betätigungshebels (12) gesichert ist.

9. Betätigungseinrichtung (40) nach Anspruch 4, wobei der Grundkörper (18) einen distalen und einen proximalen Stößel (52, 54) umfasst, und wobei der distale Stößel (52) des Grundkörpers (18) distal in Bezug auf das Lagerungsglied (44) am Grundkörper (18) angeordnet ist, und der proximale Stößel (54) proximal in Bezug auf das Lagerungsglied (44) am Grundkörper (18) angeordnet ist.

10. Betätigungseinrichtung (40) nach Anspruch 9, wobei der distale Stößel (52) die distale Führungskurve (46) in einem jeweiligen Kontaktpunkt kontaktiert und der proximale Stößel (54) die proximale Führungskurve (48) in einem jeweiligen Kontaktpunkt kontaktiert, und die distale Führungskurve (46) derart ausgebildet ist, dass sich ein Abstand des Kontaktpunkts zur Drehachse (42) ausgehend von der ersten Stellung der Betätigungseinrichtung (40) zur zweiten Stellung vergrößert, und die proximale Führungskurve (48) derart ausgebildet ist, dass sich ein Abstand des Kontaktpunkts zur Drehachse (42) ausgehend von der ersten Stellung der Betätigungseinrichtung (40) zur zweiten Stellung verkleinert.

11. Betätigungseinrichtung (40) nach Anspruch 10, wobei die Ausbildung der Führungskurven (46, 48) derart aufeinander abstimmt sind, dass durch Zusammenwirken des distalen Stößels (52) mit der distalen Führungskurve (46) ein distales Gegenlager und durch Zusammenwirken das proximalen Stößels (54) mit der proximalen Führungskurve (48) ein proximales Gegenlager bereitgestellt wird, so dass die Drehachse (42) in jeder Stellung gegen ein selbsttätiges translatorisches Verschieben ohne Betätigen des Betätigungshebels (12) gesichert ist.

12. Betätigungseinrichtung (40) nach einem der vorhergehenden Ansprüche, wobei die Betätigungseinrichtung (40) ein Rückstellelement umfasst zum Rückstellen des Betätigungshebels (12) aus der zweiten Stellung, insbesondere der Arbeitsstellung, in die erste Stellung, insbesondere die Ruhestellung.

13. Medizinisches Instrument, **dadurch gekennzeichnet, dass** das Instrument eine Betätigungseinrichtung (40) nach einem der vorstehenden Ansprüche umfasst, wobei die Betätigungseinrichtung (40) mit an einem distalen Ende (6) des Instruments angeordneten Werkzeugelementen zusammenwirkend an einem proximalen Ende (4) des Instruments angeordnet oder ausgebildet ist, und wobei die Betätigungseinrichtung (40) zum Bewegen der Werkzeugelemente relativ zueinander ausgebildet ist.

14. Medizinisches Instrument nach Anspruch 13, wobei das Instrument in Form eines elektrochirurgischen Instruments und/oder in Form eines Klammergeräts ausgebildet ist und/oder umfasst.

## Claims

1. Actuation device (40) for a medical instrument, comprising a main body (18), which has a handle element (16) provided on the main body (18), and comprising an actuation lever (12), wherein tool elements provided at a distal end (6) of the instrument can be moved by actuating the actuation lever (12) at a proximal end (4) of the instrument from distal, out of a first position, in particular a rest position, to proximal, into a second position, in particular a working position, and/or from proximal, out of the second position, to distal, into the first position, wherein the actuation lever (12) can be rotationally moved relative to the handle element (16) of the actuation device (40), and the actuation device (40) is designed such that a rotational input movement (a) of the actuation lever (12) is converted, by a transmission member (24) that can be translationally displaced along a shaft axis (10) of the main body (18), into a translational output movement (b) in order to move the tool elements, wherein a tilt lever (22) is arranged between the actuation lever (12) and the transmission member (24), wherein the tilt lever (22) is rotatably connected to the actuation lever (12) and rotatably connected to the transmission member (24), wherein a swivel pin (42), about which the actuation lever (12) can be rotated, is mounted translationally with respect to the main body (18) such that the swivel pin (42) can be translationally displaced by the rotational input movement (a) of the actuation lever (12).

2. Actuation device (40) according to claim 1, wherein the swivel pin (42) can be translationally displaced parallel to the shaft axis (10) of the main body (18) by the rotational input movement (a) of the actuation lever (12).

3. Actuation device (40) according to claim 1 or claim 2, wherein a bearing member (44), which comprises the swivel pin (42) of the actuation lever (12), is mounted in a guide of the main body (18), in particular in a guide groove of a housing of the main body (18).

4. Actuation device (40) according to any of the preceding claims, wherein the actuation lever (12), in particular the bearing member (44) of the actuation lever (12), comprises a distal guide cam (46) and a proximal guide cam (48), and the guide cams (46, 48) slide along at least one slide (50) provided on the main body (18) upon actuation of the actuation lever (12).

5. Actuation device (40) according to claim 4, wherein the guide cams (46, 48) are formed by a guide groove (68), which is in particular curved, and the slide (50) is arranged in the guide groove (68).

6. Actuation device (40) according to claim 5, wherein the translational displacement of the swivel pin (42) takes place depending on a shape of the guide groove (68), which is in particular curved.

7. Actuation device (40) according to claim 5 or claim 6, wherein the slide contacts the guide groove at a particular contact point, and a shape of the guide groove (68) is formed, with respect to the swivel pin (42), over the course of the guide groove (68) such that a distance from a particular contact point to the swivel pin increases from the first position of the actuation device (40) to the second position.

8. Actuation device (40) according to any of claims 5 to 7, wherein, by interaction of the slide (50) and the guide groove (68), a distal counter bearing and a proximal counter bearing are provided, and therefore the swivel pin (42) is secured in any position against automatic translational displacement without actuation of the actuation lever (12).

9. Actuation device (40) according to claim 4, wherein the main body (18) comprises a distal and a proximal slide (52, 54), and wherein the distal slide (52) of the main body (18) is arranged on the main body (18) distally relative to the bearing member (44), and the proximal slide (54) is arranged on the main body (18) proximally relative to the bearing member (44).

10. Actuation device (40) according to claim 9, wherein the distal slide (52) contacts the distal guide cam (46) at a particular contact point, and the proximal slide (54) contacts the proximal guide cam (48) at a particular contact point, and the distal guide cam (46) is formed such that a distance from the contact point to the swivel pin (42) increases starting from the first position of the actuation device (40) to the second position, and the proximal guide cam (48) is designed such that a distance from the contact point to the swivel pin (42) decreases starting from the first position of the actuation device (40) to the second position.

11. Actuation device (40) according to claim 10, wherein the guide cams (46, 48) are designed to correspond to one another such that, by interaction of the distal slide (52) with the distal guide cam (46), a distal counter bearing is provided, and, by interaction of the proximal slide (54) with the proximal guide cam (48), a proximal counter bearing is provided, and therefore the swivel pin (42) is secured in any position against automatic translational displacement without actuation of the actuation lever (12).

12. Actuation device (40) according to any of the preceding claims, wherein the actuation device (40) comprises a return element for returning the actuation lever (12) from the second position, in particular the working position, to the first position, in particular the rest position.

13. Medical instrument, **characterized in that** the instrument comprises an actuation device (40) according to any of the preceding claims, the actuation device (40) being arranged or designed so as to have tool elements, which are arranged at a distal end (6) of the instrument and interact at a proximal end (4), and the actuation device (40) being designed to move the tool elements relative to one another.

14. Medical instrument according to claim 13, wherein the instrument is in the form of and/or comprises an electrosurgical instrument and/or is in the form of and/or comprises a stapling device.

## Revendications

1. Dispositif d'actionnement (40) pour un instrument médical, comprenant un corps de base (18) portant, sur le corps de base (18), un élément de préhension (16) et un levier d'actionnement (12), dans lequel, par l'actionnement du levier d'actionnement (12) à l'extrémité proximale (4) de l'instrument, depuis le côté distal, d'une première position, en particulier d'une position de repos, vers le côté proximal jusqu'à une deuxième position, en particulier d'une position de travail, et/ou depuis le côté proximal de la deuxième position vers le côté distal jusqu'à la première position, des éléments d'outil prévus à l'extrémité distale (6) de l'instrument sont mobiles, le levier d'actionnement (12) étant mobile en rotation par rapport à l'élément de préhension (16) du dispositif d'actionnement (40), et le dispositif d'actionnement (40) étant conçu de sorte qu'un mouvement d'entrée rotatif (a) du levier d'actionnement (12) est, au moyen d'un organe de transmission (24) déplaçable de façon translationnelle le long d'un axe de la tige (10) du corps de base (18), converti en un mouvement de sortie translationnel (b) pour déplacer les éléments d'outil, un levier basculant (22) étant disposé entre le levier d'actionnement (12) et l'organe de transmission (24), le levier basculant (22) étant articulé de façon rotative avec le levier d'actionnement (12) et avec l'organe de transmission (24), et un axe de rotation (42), autour duquel le levier d'actionnement (12) est mobile en rotation, étant monté de façon translationnelle par rapport au corps de base (18), de sorte que, par le mouvement d'entrée rotatif (a) du levier d'actionnement (12), l'axe de rotation (42) est déplaçable de façon translationnelle.

2. Dispositif d'actionnement (40) selon la revendication 1, dans lequel, par le mouvement d'entrée rotatif (a) du levier d'actionnement (12), l'axe de rotation (42) est déplaçable de façon translationnelle parallèlement à l'axe de la tige (10) du corps de base (18).

3. Dispositif d'actionnement (40) selon l'une des revendications 1 ou 2, dans lequel un élément de palier (44) englobant l'axe de rotation (42) du levier d'actionnement (12) est logé dans un guidage du corps de base (18), en particulier dans une rainure de guidage d'un boîtier du corps de base (18).

4. Dispositif d'actionnement (40) selon l'une quelconque des revendications précédentes, dans lequel le levier d'actionnement (12), en particulier l'élément de palier (44) du levier d'actionnement (12), comprend une courbe de guidage distale (46) et une courbe de guidage proximale (48), et les courbes de guidage (46, 48) glissent, lors de l'actionnement du levier d'actionnement (12), le long d'au moins un poussoir (50) disposé sur le corps de base (18).

5. Dispositif d'actionnement (40) selon la revendication 4, dans lequel les courbes de guidage (46, 48) sont formées par une rainure de guidage (68), en particulier courbée, et le poussoir (50) est disposé dans la rainure de guidage (68).

6. Dispositif d'actionnement (40) selon la revendication 5, dans lequel le déplacement translationnel de l'axe de rotation (42) dépend de la forme de la rainure de guidage (68), en particulier de sa courbure.

7. Dispositif d'actionnement (40) selon l'une des revendications 5 ou 6, dans lequel le poussoir contacte la rainure de guidage en un point de contact respectif et la forme de la rainure de guidage (68), par rapport à l'axe de rotation (42) sur le parcours de la rainure de guidage (68), est conçue de sorte que la distance d'un point de contact respectif à l'axe de rotation augmente en passant de la première position du dispositif d'actionnement (40) à la deuxième position.

8. Dispositif d'actionnement (40) selon l'une des revendications 5 à 7, dans lequel, par la coopération du poussoir (50) et de la rainure de guidage (68), est fourni un contre-palier distal et un contre-palier proximal, de sorte que l'axe de rotation (42) est sécurisé dans chaque position contre un déplacement translationnel autonome sans actionner le levier d'actionnement (12).

9. Dispositif d'actionnement (40) selon la revendication 4, dans lequel le corps de base (18) comprend un poussoir distal et un poussoir proximal (52, 54), le poussoir distal (52) du corps de base (18) étant disposé distalement par rapport à l'élément de palier (44) sur le corps de base (18), et le poussoir proximal (54) étant disposé proximalement par rapport à l'élément de palier (44) sur le corps de base (18).

10. Dispositif d'actionnement (40) selon la revendication 9, dans lequel le poussoir distal (52) contacte la courbe de guidage distale (46) en un point de contact respectif et le poussoir proximal (54) contacte la courbe de guidage proximale (48) en un point de contact respectif, la courbe de guidage distale (46) étant conçue de sorte que la distance du point de contact à l'axe de rotation (42) augmente en passant de la première position du dispositif d'actionnement (40) à la deuxième position, et la courbe de guidage proximale (48) étant conçue de sorte que la distance du point de contact à l'axe de rotation (42) diminue en passant de la première position du dispositif d'actionnement (40) à la deuxième position.

11. Dispositif d'actionnement (40) selon la revendication 10, la configuration des courbes de guidage (46, 48) étant coordonnée de manière à ce que, par l'interaction du poussoir distal (52) avec la courbe de guidage distale (46), soit fourni un contre-appui distal, et par l'interaction du poussoir proximal (54) avec la courbe de guidage proximale (48), soit fourni un contre-appui proximal, de sorte que l'axe de rotation (42) est sécurisé dans chaque position contre un déplacement translationnel involontaire sans actionner le levier d'actionnement (12).

12. Dispositif d'actionnement (40) selon l'une des revendications précédentes, le dispositif d'actionnement (40) comprenant un élément de rappel destiné à ramener le levier d'actionnement (12) de la deuxième position, en particulier de la position de travail, à la première position, en particulier à la position de repos.

13. Instrument médical, **caractérisé en ce que** l'instrument comprend un dispositif d'actionnement (40) selon l'une des revendications précédentes, le dispositif d'actionnement (40) étant disposé ou formé à l'extrémité proximale (4) de l'instrument et agissant en coopération avec des éléments d'outil disposés à l'extrémité distale (6) de l'instrument, et le dispositif d'actionnement (40) étant conçu pour déplacer les éléments d'outil les uns par rapport aux autres.

14. Instrument médical selon la revendication 13, l'instrument étant réalisé et/ou comprenant un instrument électrochirurgical et/ou un appareil à agrafes.
